# EUROPEAN PATENT APPLICATION

(11) **EP 4 810 612 A1**
(43) Date of publication of application: **23.09.2026**
(21) Application number: 24890520.0
(22) Date of filing: 04.11.2024
(51) Int. Cl.: C12N 15/29, C12N 15/113, C12N 15/84

(54) **LATE BLIGHT RESISTANCE GENE, BIOMATERIAL, AND USE**

(30) Priority: 15.11.2023 CN 202311524783
(71) Applicant: Agricultural Genomics Institute at Shenzhen, Chinese Academy of Agricultural Sciences, Shenzhen, Guangdong 518124 (CN); Agricultural Genomics Institute, Chinese Academy of Agricultural Sciences, Shenzhen, Guangdong 518124 (CN)
(72) Inventor: HUANG, Sanwen, Shenzhen, Guangdong 518124 (CN); DONG, Suomeng, Shenzhen, Guangdong 518124 (CN); WANG, Luyao, Shenzhen, Guangdong 518124 (CN); ZHANG, Chunzhi, Shenzhen, Guangdong 518124 (CN); LI, Yuying, Shenzhen, Guangdong 518124 (CN); WANG, Pei, Shenzhen, Guangdong 518124 (CN)
(74) Representative: Murgitroyd & Company
(86) International application number: PCT/CN2024/129626
(87) International publication number: WO 2025/103163

(57) **Abstract**

The present invention relates to the technical fields of gene segregation and plant improvement, and in particular to a late blight resistance gene, a biomaterial, and a use. An Rpi-caj 1 nucleic acid molecule comprises: a nucleotide sequence as shown in any one of SEQ ID NOs: 1-6; a nucleotide sequence encoding the amino acid sequence shown in any one of SEQ ID NOs: 7-12; a nucleotide sequence shown in any one of SEQ ID NOs: 13-18; and a nucleotide sequence having at least 75% sequence identity with the described nucleotide sequences. An important gene resource is provided for late blight resistance breeding of potatoes, thereby solving the problem of loss of disease resistance of potato varieties and providing a new guarantee for persistent disease resistance of potatoes.

## Description

### TECHNICAL FIELD

This invention relates to the fields of gene isolation and plant improvement technology, particularly to a late blight resistance gene, biomaterial and use thereof.

### BACKGROUND

Potato late blight is internationally recognized as the most serious disease in potato production. Its outbreak once caused the infamous "Great Irish Famine". For more than 100 years, scientists from various countries have been continuously fighting against the rapidly mutating late blight pathogen *Phytophthora sp*. Because the pathogen causing late blight has a strong ability to mutate in the field, it may quickly overcome the resistance of most existing potato varieties, and the problem of loss of disease resistance in potato varieties is becoming increasingly serious, posing a serious threat to potato production. As the world's largest potato producer, China relies heavily on agricultural chemicals for the prevention and control of late blight. The extensive use of agricultural chemicals not only increases production costs and pollutes the environment, but also poses a threat to people's life and health. Moreover, due to the long-term and extensive use of fungicides such as metalaxyl, the physiological races of late blight have been constantly mutating and have gradually developed fungicide tolerance. Therefore, breeding and planting varieties with long-lasting resistance to late blight is an effective alternative to control this disease. Discovering new broad-spectrum resistance genes and appropriately pyramiding multiple resistance genes is the top priority of current breeding work for potato disease resistance.

The discovery and cloning of new broad-spectrum resistance genes in potatoes is a hot topic in late blight research. In early research on late blight resistance breeding, the initial resistance genes mainly came from the Mexican hexaploid wild species *Solanum demissum*, which includes 11 (*R1*-*R11*) late blight resistance locus, all of which are variety-specific major resistance genes. However, with the continuous mutation of various physiological races of *Phytophthora sp.*, the early disease-resistant genes have all lost their disease-resistant function. With the rapid development of modern molecular biology and high-throughput sequencing technology, potato breeders in Europe and America are constantly cloning new disease-resistant genes from various wild potato resources by using map-based cloning of disease-resistant genes combined with comparative genomics, resistance gene enrichment sequencing (RenSeq), and pathogen effector omics. The most well-known broad-spectrum resistance genes include *Rpi-vnt1.1*, *Rpi-blb1*, *R8*, and *Rpi-amr1*. However, pathogenic races of *Phytophthora sp*. that can overcome these resistance genes have also emerged. Therefore, it is urgent to continue to discover and clone new broad-spectrum resistance genes for potatoes.

### SUMMARY

In view of this, this invention provides a new late blight resistance gene, biomaterial, and use thereof. This invention provides a newly discovered late blight resistance gene Rpi-caj1, which provides an important genetic resource for potato breeding for resistance to late blight.

To achieve the above object, this invention provides the following technical solutions.

This invention provides an Rpi-caj1 nucleic acid molecule including at least one polynucleotide selected from the group consisting of:
(a1) any one of the polynucleotides represented by SEQ ID NOs: 1-6;
(a2) a polynucleotide encoding any one of the amino acid sequences represented by SEQ ID NOs: 7-12, and optionally the polynucleotide is not naturally occurring;
(a3) any one of the polynucleotides represented by SEQ ID NOs: 13-18;
(a4) a polynucleotide having at least 75% sequence identity with any one of the polynucleotides shown in (a1) and (a3); where the nucleic acid molecule is capable of conferring resistance to a plant disease caused by at least one race of at least one *Phytophthora sp*. to a plant comprising the nucleic acid, and optionally where the polynucleotide is not naturally occurring; and
(a5) a polynucleotide encoding an amino acid sequence having at least 75% sequence identity with any one of the amino acid sequences shown in (a2), where the nucleic acid molecule is capable of conferring resistance to a plant disease caused by at least one race of at least one *Phytophthora sp*. to a plant comprising the nucleic acid, and optionally where the polynucleotide is not naturally occurring.

Optionally, the above nucleic acid molecule is a synthetic and/or an isolated nucleic acid molecule.

This invention has identified wild diploid potato materials of *Solanum cajamarquense*, which exhibit strong resistance to late blight, through extensive experiments. Whole-genome sequencing and evolutionary analysis were performed on these materials, leading to the discovery of the candidate gene Rpi-caj1. The Rpi-caj1-C534 gene, firstly discovered in this invention, is originated from wild diploid potato materials of *Solanum cajamarquense*. Based on this gene sequence, genes Rpi-caj1-C813, Rpi-caj1-C550, Rpi-caj1-C509, Rpi-caj1-C450, and Rpi-caj1-C419 were subsequently discovered in *Solanum cajamarquense*, *Solanum sogarandinum*, *Solanum cardiophyllum*, *Solanum piurae*, and *Solanum candolleanum* materials in this invention. Unlike many known late blight resistance genes, the amino acid sequences encoded by the above genes all include a TIR domain and an NB-ARC domain. These TNL genes are named as Rpi-caj1 (or Rpi-cjm1).

In this invention, the amino acid sequence encoded by the above Rpi-caj1 gene includes three domains, i.e., TIR (Toll-like/Interleukin 1 receptor) domain: amino acids 8-185; NBS (nucleotide-binding site) domain: amino acids 203-433; and LRR (Leucine-rich repeats) domain: amino acids 433-606.

In (a4) above, exemplary polynucleotides having at least 75% sequence identity are polynucleotides having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% identity, preferably polynucleotides having 97.0%-98.0%, 97.5%-98.5%, 98.0%-99.0%, 98.5%-99.5%, or 99.0%-100% identity.

In (a5) above, exemplary polynucleotides encoding amino acid sequences having at least 75% sequence identity are polynucleotides encoding amino acid sequences having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% identity, preferably polynucleotides encoding amino acid sequences having 97.0%-98.0%, 97.5%-98.5%, 98.0%-99.0%, 98.5%-99.5%, or 99.0%-100% identity.

In some embodiments, the natural polynucleotide of the nucleic acid molecule related to this invention may be a variant sequence derived from different species and/or individual plants of *Solanum*, and its artificial nucleotide sequence (or artificial variant sequence) may be a variant sequence obtained by appropriately modifying the natural nucleotide sequence. The modifications include, but are not limited to: appropriate nucleotide substitution/addition/deletion, truncation of N-terminal amino acids, codon optimization suitable for host cell preferences, addition of a tag, fusion, and other corresponding nucleotide variant sequences that do not affect the biological activity of the target protein.

Preferably, the polynucleotide of Rpi-caj1 in this invention is at least one of the polynucleotides shown in (a1)-(a3) and the artificial variant sequences thereof.

In a particular embodiment of this invention, the Rpi-caj1 nucleic acid molecule includes at least one polynucleotide selected from the group consisting of:
(a1) the polynucleotide represented by SEQ ID NO: 1;
(a2) a polynucleotide encoding the amino acid sequence represented by SEQ ID NO: 7;
(a3) the polynucleotide represented by SEQ ID NO: 13;
(a4) a polynucleotide having at least 90% sequence identity with any one of the polynucleotides shown in (a1) and (a3); and
(a5) a polynucleotide encoding any one of the amino acid sequences having at least 90% sequence identity with the amino acid sequence shown in (a2).

In a particular embodiment of this invention, where in (a4), the polynucleotide having at least 90% identity with the polynucleotide represented by SEQ ID NO: 1 includes at least one of the polynucleotides represented by SEQ ID NOs: 2-6.

In a particular embodiment of this invention, where in (a4), the polynucleotide having at least 90% identity with the polynucleotide represented by SEQ ID NO: 13 includes at least one of the polynucleotides represented by SEQ ID NOs: 14-18.

In a particular embodiment of this invention, where in (a5), the polynucleotide encoding an amino acid sequence having at least 90% sequence identity with the amino acid sequence represented by SEQ ID NO: 7 includes at least one of the polynucleotides represented by SEQ ID NOs: 8-12.

The Rpi-caj1 polynucleotide of this invention includes, but is not limited to: a polynucleotide including a natural promoter and wild-type Rpi-caj1-C534, Rpi-caj1-C813, Rpi-caj1-C550, Rpi-caj1-C509, Rpi-caj1-C450, and Rpi-caj1-C419 genes of a 3' neighboring region including the coding region, the cDNA sequence, and a polynucleotide including only the coding region.

This invention also provides a nucleic acid molecule composition including the above nucleic acid molecule and an additional resistance (R) gene;
optionally, the additional *R* gene includes, but is not limited to at least one of the following genes: Rpi-amr3i (accession number KT373889; SEQ ID NO: 1 of WO2016/182881), Rpi-blb1 (also known as "RB"; accession numbers FB764493.1 and AY336128.1), Rpi-sto1 (accession number EU884421), Rpi-pta1 (accession number EU884422), Rpi-blb2 (accession number DQ122125), Rpi-blb3 (accession number FJ536326), Rpi-abpt (accession number FJ536324), R2-like (accession number FJ536323), R2 (accession number FJ536325), Rpi-edn1.1 (accession number GU563963), Rpi-edn1.2, Rpi-snk1.1, Rpi-snk1.2, Rpi-hjt1.1-Rpi-hjt1.3 (accession numbers GU563971-3), Rpi-bt1 (accession number FJ188415), R1 (accession number AF447489), R3a (accession number AY849382), R3b (accession number JF900492), Rpi-vnt1.1 (accession number FJ423044), Rpi-vnt1.2 (accession number FJ423045), Rpi-vnt1.3 (accession number FJ423046), Rpi-mcq1 (accession number GN043561), Rpi-chc, Ph-3 (accession number KJ563933), and R8 (accession number KU530153).

Polynucleotides corresponding to the accession numbers of the genes listed above or any genes or proteins disclosed elsewhere in this document are available from publicly available online nucleotide and amino acid sequence databases, such as GenBank and EMBL databases (available on the World Wide Web at ncbi.nlm.nih.gov/genbank and ebi.ac.uk, respectively).

This invention also provides a biomaterial, which is any one selected from the following (b1) to (b3):
(b1) an expression cassette or expression cassette composition including the above nucleic acid molecule or nucleic acid molecule composition;
(b2) a vector or vector composition including the above nucleic acid molecule or nucleic acid molecule composition, or a vector or vector composition including the expression cassette or expression cassette composition described in (b1); and
(b3) a host cell including the above nucleic acid molecule or nucleic acid molecule composition, or a host cell including the expression cassette or expression cassette composition described in (b1), or a host cell including the vector or vector composition described in (b2).

Optionally, the host cell may include bacterial or fungal cell.

In one embodiment of this invention, the biomaterial is an expression cassette or expression cassette composition.

In the embodiments of this invention, when the above nucleic acid molecule or nucleic acid molecule composition includes at least two nucleic acid molecules, it may be prepared into an expression cassette or into a composition consisting of at least two expression cassettes.

In embodiments of this invention, the expression cassette or expression cassette composition further includes a regulatory element, which includes at least one selected from the group consisting of: a promoter, an enhancer, a leader sequence, a transposon, a terminator, and a marker gene.

In embodiments of this invention, the expression cassette or expression cassette composition further includes an operatively connected promoter.

Optionally, the promoter includes an operatively connected endogenous promoter and/or an operatively connected heterogeneous promoter.

Optionally, the operatively connected endogenous promoter is a natural promoter of the Rpi-caj1 gene, such as the endogenous promoter Pcaj.

In embodiments of this invention, the endogenous promoter Pcaj includes at least one polynucleotide selected from the group consisting of: any one of the polynucleotides represented by SEQ ID NOs: 19-24, and/or a polynucleotide having at least 75% sequence identity with any one of the polynucleotides represented by SEQ ID NOs: 19-24. Exemplary polynucleotides having at least 75% sequence identity are polynucleotides having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity.

In embodiments of this invention, the endogenous promoter Pcaj includes at least one of the following polynucleotides: the polynucleotide represented by SEQ ID NO: 19, and/or a polynucleotide having at least 75% sequence identity with the polynucleotide represented by SEQ ID NO: 19.

In embodiments of this invention, the polynucleotide having at least 75% sequence identity with the polynucleotide represented by SEQ ID NO: 19 includes at least one of the polynucleotides represented by SEQ ID NOs: 20-24.

In embodiments of this invention, the selection of the operatively connected heterogeneous promoter may depend on many factors, such as the desired timing, location and expression pattern, and responsiveness to specific biological or abiotic stimuli. Optionally, the operatively connected heterogeneous promoter includes at least one selected from the group consisting of: a pathogen-inducible promoter, a constitutive promoter, a tissue-preferred promoter, a wound-inducible promoter, and a chemically regulated promoter. For example, the pblb3 promoter (Lokossou A A et al. (2009) Molecular plant-microbe interactions, 22(6):630-641). ); core CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (US Patent No. 5,659,026), etc. The preferred promoter is one that is suitable for the stable expression of the same types of disease resistance genes in *Solanum* crops.

The exact nature of the regulatory sequences required for gene expression may vary between species or cell types, but generally should include 5' non-transcriptional and 5' non-translational sequences for transcription and translation initiation, such as a TATA box, a capped sequence, a CAAT sequence, etc., as needed. In particular, this 5' non-transcriptional regulatory sequence will include a promoter region, which includes a promoter sequence that controls transcriptional control of the effectively linked gene. The regulatory sequence may also include an enhancer sequence or a desired upstream activator sequence. The expression cassette of this invention may optionally include a 5' leader or signal sequence.

In the embodiments of this invention, the expression of the nucleic acid molecule may be regulated by manipulating the copy number of genes or operons in cells.

In some embodiments, the expression of the nucleic acid molecule may be regulated by the order of nucleic acid molecule within the operating module.

In some embodiments, the expression of the nucleic acid molecule is regulated by integrating one or more nucleic acid molecules or operons into the chromosome.

In another embodiment of this invention, the biomaterial is a vector or a vector composition.

In some embodiments, the biomaterial is a vector or vector composition including the above nucleic acid molecule or nucleic acid molecule composition.

In some embodiments, the biomaterial is a vector or vector composition including the above-described expression cassette or expression cassette composition.

In some embodiments, one or more nucleic acid molecules related to this invention are expressed in an expression vector. The "vector" used herein may be any one of a large number of nucleic acids, in which one or more desired sequences may be inserted through restriction enzyme digestion and ligation, so as to be transported in different genetic environments or expressed in host cells. Vectors are usually composed of DNA, but can also be composed of RNA.

In the embodiments of this invention, the vector includes plasmid, chloroplast, viral vector, bacteriophage, phagemid, cosmid, F cosmid, bacterial phage, or artificial chromosome; and optionally, the viral vector includes adenovirus vector, retrovirus vector, or adeno-associated virus vector; and optionally, the vector includes bacterial artificial chromosome (BAC), plasmid, bacterial phage P1-derived vector (PAC), yeast artificial chromosome (YAC), or mammalian artificial chromosome (MAC). For example, the vector includes pFastBac1, pYES2, pYES2.1, pESC-Ura, pESC-Trp, pESC-Leu, pESC-His, pGEX2T, pTAex3, pUSA, pYMB0, pHT43, pET28b, pIJ702, pUCP19, pYMB03, pHT43, pEAQ, pBin307, pPZP, pSAT, pCAMIA-1300, etc.

A cloning vector can autonomously replicate or integrate into the host cell genome. It is also characterized by one or more restriction endonuclease sites, which may be used to cut the vector in a defined manner and to ligate the desired DNA sequence into the vector, so that the new plasmid retains its ability to replicate in the host cell. When the vector is a plasmid, the replication of the desired sequence can occur multiple times as the number of copies of the plasmid in the host cell (such as a bacterial host) increases, or it can occur only once in each host before the host reproduces by mitosis. When the vector is a bacteriophage, replication can occur actively during the lysis phase or passively during the lysogenic phase.

Desired DNA sequence can be inserted into an expression vector through restriction enzyme digestion and ligation, so that the desired DNA sequence is effectively linked with a regulatory sequence and may be expressed as an RNA transcript. The vector may also include one or more marker sequences suitable for identifying whether cells have been transformed or transfected by the vector. The marker includes a gene encoding a protein that increases or decreases resistance or sensitivity to antibiotics or other compounds, a gene encoding an enzyme whose activity may be detected by a standard method known in the art (e.g., β-galactosidase, luciferase, or alkaline phosphatase), and a gene having a visible effect on the phenotype of transformed or transfected cells, hosts, colonies, or plaques (e.g., green fluorescent protein). The preferred vector is one that can autonomously replicate and express the structural gene products present in the DNA fragment to which it is effectively linked.

In the embodiments of this invention, when the nucleic acid molecule or the nucleic acid molecule composition includes at least two nucleic acid molecules, it may be prepared as a single vector or as a composition consisting of at least two vectors.

In another embodiment of this invention, the biomaterial is a host cell.

In some embodiments, the biomaterial is a host cell including the above nucleic acid molecule or nucleic acid molecule composition.

In some embodiments, the biomaterial is a host cell including the above expression cassette or expression cassette composition.

In some embodiments, the biomaterial is a host cell including the above vector or vector composition.

Optionally, the host cell may include bacterial or fungal cell.

This invention also provides a promoter for driving the transcription of the Rpi-caj1 gene, where the promoter Pcaj includes at least one of the following polynucleotides: any one of the polynucleotides represented by SEQ ID NOs: 19-24, and/or, a polynucleotide having at least 75% sequence identity with any one of the polynucleotides represented by SEQ ID NOs: 19-24. Exemplary polynucleotides having at least 75% sequence identity are polynucleotides having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity.

In embodiments of this invention, the endogenous promoter Pcaj includes at least one of the following polynucleotides: the polynucleotide represented by SEQ ID NO: 19, and/or a polynucleotide having at least 75% sequence identity with the polynucleotide represented by SEQ ID NO: 19.

In the embodiments of this invention, the polynucleotide having at least 75% sequence identity with the polynucleotide represented by SEQ ID NO: 19 includes at least one of the polynucleotides represented by SEQ ID NOs: 20-24.

This invention provides a method for generating a host cell, the method including transforming the host cell with at least one of the above nucleic acid molecule or nucleic acid molecule composition, expression cassette or expression cassette composition, and vector or vector composition.

This invention provides use of any one of the nucleic acid molecule, nucleic acid molecule composition, and biomaterial according to this invention in enhancing the resistance of a plant to a plant disease caused by at least one race of at least one *Phytophthora sp*.

In the embodiments of this invention, the plants include solanaceous plants.

Optionally, the solanaceous plants include at least one selected from the group consisting of: potato, tomato, eggplant, pepper, tobacco, petunia, tomatillo (*Physalis ixocarpa*), and cape gooseberry (*Physalis peruviana*).

Preferably, the solanaceous plants are potato and/or tomato.

This invention also provides a method for enhancing a plant's resistance to a plant disease caused by at least one race of at least one *Phytophthora sp.*, where the method includes modifying at least one plant cell to include a heterologous polynucleotide including the above nucleic acid molecule or nucleic acid molecule composition.

In a preferred embodiment of this invention, the species of *Phytophthora* is *Phytophthora infestans*. In other embodiments, a species of *Phytophthora* is such a species of *Phytophthora* that can cause a plant disease on at least one plant. For this invention, certain species of *Phytophthora* include, but are not limited to: *Phytophthora parasitica, Phytophthora ramorum, Phytophthora ipomoeae, Phytophthora mirabilis, Phytophthora capsici, Phytophthora porri, Phytophthora sojae, Phytophthora palmivora*, and *Phytophthora phaseoli*.

In embodiments of this invention, compared to a control plant (a control plant that does not include the heterologous polynucleotide), a plant including the heterologous polynucleotide have enhanced resistance to a plant disease caused by at least one race of at least one *Phytophthora sp*.

In the embodiments of this invention, modifying at least one plant cell to include a heterologous polynucleotide particularly involves: transforming at least one of the nucleic acid molecule, nucleic acid molecule composition, or expression cassette or expression cassette composition, vector or vector composition in a biomaterial into plant cells, thereby enabling the plant cells to express proteins.

In the above methods, the methods for transforming the nucleic acid molecule, nucleic acid molecule combination, expression cassette, and vector into plant cells include, but are not limited to, *Agrobacterium*-mediated transformation, gene gun transformation, electroporation, polyethylene glycol (PEG) transformation, lipid transfection, heat shock, calcium phosphate precipitation, virus-mediated transformation, microinjection, and gene editing technology.

In a particular embodiment of this invention, the method for expressing a protein is: for example, (1) constructing a vector including the nucleic acid molecule or nucleic acid molecule composition of this invention; (2) transforming the obtained vector into a plant cell; and (3) culturing the obtained plant cell to express the gene and generate the protein.

In embodiments of this invention, the method for enhancing plant resistance further includes regenerating the plant cell into a plant whose genome includes the heterologous polynucleotide.

Preferably, compared with the resistance of a control plant to a plant disease, the regenerated plant includes enhanced resistance to a plant disease caused by at least one race of at least one *Phytophthora sp*.

Preferably, compared with a control plant, a plant including the heterologous polynucleotide has enhanced resistance to a plant disease caused by at least two, three, four, five, six, seven, eight, nine, ten, eleven, twelve, thirteen, fourteen, fifteen, sixteen or more races of *Phytophthora sp*.

In this embodiment of the invention, compared with a control plant, a plant whose genome includes the heterologous polynucleotide has enhanced or increased resistance by at least 25% to a plant disease caused by at least one race of at least one *Phytophthora sp*. For example, increasing or adding at least 25%, at least 50%, at least 75%, at least 100%, at least 150%, at least 200%, at least 250%, at least 300%, at least 350%, at least 400%, at least 450%, at least 500%, or more.

In some embodiments, a plant disease caused by at least one race of at least one *Phytophthora sp*. is called late blight.

Depending on the desired outcome, the heterologous polynucleotide of this invention may be stably integrated into the genome of a plant cell or unstablely integrated into the genome of a plant cell.

For example, if the desired outcome is to produce a plant with enhanced resistance to a plant disease caused by at least one race of at least one *Phytophthora sp.*, then a heterologous polynucleotide may be fused, for example, into a plant transformation vector suitable for stably integrating the heterologous polynucleotide into the genome of the plant cell. In some embodiments, the nucleic acid molecule or nucleic acid molecule composition (heterologous polynucleotides) of this invention may be stably integrated into the genome of a host cell. A plant with this stable transformation can pass on the heterologous polynucleotides to subsequent generations of offspring plants through sexual and/or asexual reproduction.

In the embodiments of this invention, the modification of at least one plant cell to include a heterologous polynucleotide includes genome editing technology.

In this invention, where it is not desired to stably integrate the heterologous polynucleotide into the genome of a plant, a transient conversion method may be used to introduce the heterologous polynucleotide into one or more plant cells of the plant. In other embodiments, modified viruses and/or modified viral nucleic acids are applied to a plant or parts thereof via microinjection, particle bombardment, viral vector infection, or by spraying, irrigation, or dusting, thereby unstablely integrating the heterologous polynucleotide into the genome of a host cell, and enabling the host or host cells to transiently express the target gene.

In the embodiments of this invention, the plant includes any plant species, such as monocots, dicots, and conifers.

In the particular embodiment, the plants in this invention include crop plants such as corn, soybean, wheat, rice, cotton, alfalfa, sunflower, brassica (certain species of the genus *Brassica*, in particular *Brassica napus*, *Brassica rapa*, and *Brassica juncea*), canola (*Brassica napus*), sorghum, millet, barley, triticale, safflower, peanut, sugarcane, tobacco, potato, tomato, eggplant, and pepper.

Preferably, the plant includes solanaceous plants.

Preferably, the solanaceous plants include, but are not limited to, at least one selected from the group consisting of: potato, tomato, eggplant, pepper, tobacco, petunia, tomatillo (*Physalis ixocarpa*), and cape gooseberry (*Physalis peruviana*).

In a more preferred embodiment, the solanaceous plants are potato and/or tomato.

This invention also provides a method for limiting a plant disease caused by at least one race of at least one *Phytophthora sp*. in crop production, where the method includes:
modifying at least one plant cell to include a heterologous polynucleotide including the above nucleic acid molecule or nucleic acid molecule composition;
regenerating the plant cell into a plant whose genome includes the heterologous polynucleotide;
growing seedlings, tubers, or seeds of the plant, and cultivating the seedlings, tubers, or seeds under conditions conducive to the growth and development of the plant.

In embodiments of this invention, the method further includes harvesting at least one of the following from the plant: fruits, tubers, leaves, or seeds.

This invention also provides a method for detecting disease resistance of a plant, where the method includes detecting the presence of the above nucleic acid molecule or nucleic acid molecule composition in the plant, or in plant parts or plant cells thereof.

In embodiments of this invention, the disease resistance of a plant includes resistance to a plant disease caused by at least one race of at least one *Phytophthora sp*.

In some embodiments, a plant disease caused by at least one race of at least one *Phytophthora sp*. is called late blight.

In some embodiments, the method for detecting the disease resistance of a plant may be used to breed solanaceous plants resistant to a plant disease (e.g., late blight) caused by *Phytophthora sp.*. This resistant plant may be used in the agricultural production of its fruit, tubers, leaves, and/or seeds for human or animal consumption or other purposes.

In embodiments of this invention, the method for detecting the presence of the nucleic acid molecule or nucleic acid molecule composition includes: detecting the presence of the nucleic acid molecule or nucleic acid molecule composition by detecting an intact nucleic acid molecule or nucleic acid molecule composition, or by detecting at least one molecular marker in the nucleic acid molecule or nucleic acid molecule composition.

In embodiments of this invention, the means of detecting the presence of the above nucleic acid molecule or nucleic acid molecule composition includes PCR amplification, nucleic acid sequencing, nucleic acid hybridization, or an immunoassay for detecting a proteins or polypeptide encoded by the nucleic acid molecule or nucleic acid molecule composition.

In some embodiments, in the method for detecting a solanaceous plant exhibiting newly conferred or enhanced resistance to a plant disease caused by at least one race of at least one *Phytophthora sp.*, the detection of the presence of the Rpi-caj1 polynucleotide in the solanaceous plant may involve one or more of the following molecular biology techniques known in the art, including but not limited to: isolating genomic DNA and/or RNA from a plant, amplifying the nucleic acid molecule including the Rpi-caj1 polynucleotide and/or molecular marker of this invention by PCR amplification, sequencing the nucleic acid molecule including the Rpi-caj1 polynucleotide and/or molecular marker, detecting the Rpi-caj1 polynucleotide, molecular marker, or transcripts of the Rpi-caj1 polynucleotide by nucleic acid hybridization, and performing an immunological assay for detecting the R protein encoded by the Rpi-caj1 polynucleotide. Particularly, oligonucleotide probes and PCR primers may be designed to detect the Rpi-caj1 polynucleotide of this invention, and such probes and PCR primers may be used in methods disclosed elsewhere herein or known in the art to rapidly detect the presence of one or more plants including the Rpi-caj1 polynucleotide of this invention in a plant population.

In other embodiments of this invention, detecting the presence of the Rpi-caj1 polynucleotide includes detecting the presence of the R protein encoded by the Rpi-caj1 polynucleotide by immunological detection method, for example, involving an antibody specific to the R protein.

This invention also provides a method for selecting a plant, the method includes: detecting the presence of the above nucleic acid molecule or nucleic acid molecule composition in a plant, or in plant parts or plant cells thereof; and selecting a plant that includes at least one copy of the nucleic acid molecule or nucleic acid molecule composition in its genome.

In embodiments of this invention, the method for detecting the presence of the nucleic acid molecule or nucleic acid molecule composition includes: detecting the presence of the nucleic acid molecule or nucleic acid molecule composition by detecting an intact nucleic acid molecule or nucleic acid molecule composition, or by detecting at least one molecular marker in the nucleic acid molecule or nucleic acid molecule composition.

In embodiments of this invention, the method for detecting the presence of the above nucleic acid molecule or nucleic acid molecule composition includes: PCR amplification, nucleic acid sequencing, nucleic acid hybridization, or an immunological assay for detecting a proteins or polypeptide encoded by the nucleic acid molecule or nucleic acid molecule composition.

In some embodiments, in the method for detecting a solanaceous plant exhibiting newly conferred or enhanced resistance to a plant disease caused by at least one race of at least one *Phytophthora sp.*, the detection of the presence of the Rpi-caj1 polynucleotide in the solanaceous plant may involve one or more of the following molecular biology techniques known in the art, including but not limited to: isolating genomic DNA and/or RNA from the plant; amplifying nucleic acid molecule including the Rpi-caj1 polynucleotide and/or molecular marker as described herein by PCR amplification; sequencing nucleic acid molecules including the Rpi-caj1 polynucleotide and/or molecular marker; detecting the Rpi-caj1 polynucleotide, molecular marker, or transcripts of the Rpi-caj1 polynucleotide by nucleic acid hybridization; and performing an immunological assay for detecting the R protein encoded by the Rpi-caj1 polynucleotide. Particularly, oligonucleotide probes and PCR primers may be designed to detect the Rpi-caj1 polynucleotide of this invention, and such probes and PCR primers may be used in methods disclosed elsewhere herein or known in the art to rapidly detect the presence of one or more plants including the Rpi-caj1 polynucleotide of this invention in a plant population.

In this embodiment of the invention, the presence of the nucleic acid molecule or nucleic acid molecule composition may be detected by using the primers represented by SEQ ID NOs: 25-38.

In other embodiments of this invention, detecting the presence of the Rpi-caj1 polynucleotide includes detecting the presence of the R protein encoded by the Rpi-caj1 polynucleotide by using an immunological detection method, for example, involving an antibody specific to the R protein.

This invention also provides a method for introducing the above nucleic acid molecule or nucleic acid molecule composition into a plant, where the method includes:
(c1) hybridizing a first plant with a second plant to produce offspring plants; where
the first plant is a plant whose genome includes at least one copy of the above nucleic acid molecule or nucleic acid molecule composition; and
the second plant is a plant whose genome lacks the nucleic acid molecule or nucleic acid molecule composition; and
(c2) selecting an offspring plant whose genome includes at least one copy of the nucleic acid molecule or nucleic acid molecule composition.

In the embodiments of this invention, (c2) particularly refers to:
detecting the presence of the above nucleic acid molecule or nucleic acid molecule composition in the offspring plant, or in plant parts or plant cells thereof; and
selecting an offspring plant whose genome includes at least one copy of the nucleic acid molecule or nucleic acid molecule composition.

In the embodiments of this invention, the first plant and the second plant may be the same species or may be different species. This hybridization of a first species of plant with a second species of plant is called interspecific hybridization, and it may be used to introduce one or more target genes from one species into a related species that lacks one or more target genes. It usually involves backcrossing the offspring with the related species over multiple generations, and selecting offspring including one or more target genes in each generation. Such methods of interspecific hybridization, gene introgression, and backcrossing are well known in the art, and may be used in the methods described in this invention.

In the method for introducing at least one Rpi-caj1 gene of the present invention into a plant lacking the at least one Rpi-caj1 gene in its genome, the first plant or the second plant may be a pollen donor plant. For example, if the first plant is a pollen donor plant, then the second plant is a pollen recipient plant. Similarly, if the second plant is a pollen donor plant, then the first plant is a pollen recipient plant. After hybridization, the pollen recipient plant is grown under conditions favorable to plant growth and development, and for a sufficient period of time to allow the seeds to mature or to achieve other desired growth stages for subsequent in vitro germination processes (such as embryo rescue). Seeds can then be harvested and those including the Rpi-caj1 gene may be detected by any method known in the art, including, for example, methods described elsewhere herein for detecting a solanaceous plant exhibiting newly conferred or enhanced resistance to a plant disease caused by at least one race of at least one *Phytophthora sp*. In some embodiments, the first plant is a potato plant including one or more Rpi-caj1 genes, and the second plant is a potato plant lacking the one or more Rpi-caj1 genes.

This invention also provides a plant or plant parts thereof, where the plant is any one of the following:
(d1) a plant including at least one of the above nucleic acid molecule, nucleic acid molecule composition, expression cassette, vector, or host cell;
(d2) a plant formed by the growth of a plant cell modified to include the above nucleic acid molecule or nucleic acid molecule composition;
(d3) a plant produced by the above method for enhancing resistance to a plant disease caused by at least one race of at least one *Phytophthora sp.*;
(d4) the offspring formed by self-pollination of any one of the plants in (d1)-(d3), and the plants formed by the growth of the offspring; and
(d5) the offspring formed by hybridization of any one of the plants in (d1)-(d3) with other varieties, and the plants formed by the growth of the offspring; preferably, the hybridization is carried out by any one of the above methods for introducing the above nucleic acid molecule or nucleic acid molecule composition into a plant.

The plant parts mentioned above are roots, stems, tubers, leaves, flowers, fruits, pollen, or seeds.

As for the plant including nucleic acid molecule and nucleic acid molecule composition (heterologous polynucleotides) provided herein, the heterologous polynucleotide includes the polynucleotide of *R* gene according to this invention. Preferably, the polynucleotide of this *R* gene encodes the full-length R protein of this invention, or at least one or more functional portions or one or more domains thereof.

The plants disclosed herein may be used in agricultural crop production, particularly in areas where such plant diseases are prevalent and are known to have a negative impact or at least a potential negative impact on agricultural yields, for limiting a plant disease caused by at least one race of at least one *Phytophthora sp*.

In a particular embodiment, the plants in this invention include crop plants such as corn, soybean, wheat, rice, cotton, alfalfa, sunflower, brassica (certain species of the genus *Brassica*, in particular *Brassica napus*, *Brassica rapa*, and *Brassica juncea*), canola (*Brassica napus*), sorghum, millet, barley, triticale, safflower, peanut, sugarcane, tobacco, potato, tomato, eggplant, and pepper.

Preferably, the plant includes solanaceous plants.

Preferably, the solanaceous plants include, but are not limited to, at least one selected from the group consisting of: potato, tomato, eggplant, pepper, tobacco, petunia, tomatillo (*Physalis ixocarpa*), and cape gooseberry (*Physalis peruviana*).

In a more preferred embodiment, the solanaceous plants are potato and/or tomato.

This invention also provides a plant or plant parts thereof, the plant includes a transgenic plant including the above nucleic acid molecule or nucleic acid molecule composition.

In particular embodiments, the plant or plant parts thereof in this invention include crop plants such as corn, soybean, wheat, rice, cotton, alfalfa, sunflower, brassica (certain species of the genus *Brassica*, in particular *Brassica napus*, *Brassica rapa*, and *Brassica juncea*), canola (*Brassica napus*), sorghum, millet, barley, triticale, safflower, peanut, sugarcane, tobacco, potato, tomato, eggplant, and pepper..

In particular embodiments, the plant or plant parts thereof includes solanaceous plants. solanaceous plants include, but are not limited to, at least one of the following: potato, tomato, eggplant, pepper, tobacco, petunia, tomatillo (*Physalis ixocarpa*), and cape gooseberry (*Physalis peruviana*).

In a more preferred embodiment, the solanaceous plants are potato and/or tomato.

This invention also provides a transgenic plant or plant parts thereof, the plant includes the above nucleic acid molecule or nucleic acid molecule composition, and a promoter, where the nucleic acid molecule or nucleic acid molecule composition is operatively connected to the promoter.

Preferably, the promoter includes an endogenous promoter that is operatively connected, and/or a heterogeneous promoter that is operatively connected.

This invention also provides a method for producing a plant, the method includes transforming a plant with at least one of the following materials: the above nucleic acid molecule or nucleic acid molecule composition, expression cassette or expression cassette composition, and vector or vector composition.

This invention also provides a plant obtained by the method described above for producing a plant.

In a particular embodiment, the plants in this invention include crop plants such as corn, soybean, wheat, rice, cotton, alfalfa, sunflower, brassica (certain species of the genus *Brassica*, in particular *Brassica napus*, *Brassica rapa*, and *Brassica juncea*), canola (*Brassica napus*), sorghum, millet, barley, triticale, safflower, peanut, sugarcane, tobacco, potato, tomato, eggplant, and pepper..

Preferably, the plant includes solanaceous plants.

Preferably, the solanaceous plants include, but are not limited to, at least one selected from the group consisting of: potato, tomato, eggplant, pepper, tobacco, petunia, tomatillo (*Physalis ixocarpa*), and cape gooseberry (*Physalis peruviana*).

In a more preferred embodiment, solanaceous plants are potato and/or tomato.

This invention also provides an agricultural product for human or animal consumption, which includes the aforementioned plant or plant parts thereof, or the agricultural product for human or animal consumption is a product made from the aforementioned plant or plant parts thereof.

In the embodiments of this invention, agricultural products for human or animal consumption include food and other agricultural products. Other agricultural products include tobacco products (such as cigarettes, cigars, tobacco pipes and chewing tobacco) produced from tobacco leaves, as well as food and industrial starch products produced from potato tubers. Such food may be consumed or used by humans and other animals, where other animals include but not limited to: pets (such as dogs and cats), livestock (such as pigs, cattle, chickens, turkeys and ducks), and animals produced in freshwater or marine aquaculture systems (such as fish, shrimp, prawns, crayfish and lobsters).

This invention also provides an Rpi-caj1 protein including at least one amino acid sequence selected from the group consisting of:
(e1) any one of the amino acid sequences represented by SEQ ID NOs: 7-12;
(e2) an amino acid sequence encoded by any one of the polynucleotides represented by SEQ ID NOs: 1-6;
(e3) an amino acid sequence encoded by any one of the polynucleotides represented by SEQ ID NOs: 13-18; and
(e4) an amino acid sequence having at least 75% sequence identity with any one of the amino acid sequences represented by SEQ ID NOs: 7-12, where a protein including the amino acid sequence is capable of conferring resistance to a plant disease caused by at least one race of at least one *Phytophthora sp*.

In the above (e4), the exemplary amino acid sequences having at least 75% sequence identity are amino acid sequences having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% identity, preferably amino acid sequences having 97.0%-98.0%, 97.5%-98.5%, 98.0%-99.0%, 98.5%-99.5%, or 99.0%-100% identity.

In a particular embodiment of this invention, an Rpi-caj1 protein includes at least one amino acid sequence selected from the group consisting of:
(e1) the amino acid sequence represented by SEQ ID NO: 7;
(e2) an amino acid sequence encoded by the polynucleotide represented by SEQ ID NO: 1 or a polynucleotide having at least 90% identity with the polynucleotide represented by SEQ ID NO: 1;
(e3) an amino acid sequence encoded by the polynucleotide represented by SEQ ID NO: 13 or a polynucleotide having at least 90% identity with the polynucleotide represented by SEQ ID NO: 13;
(e4) an amino acid sequence having at least 90% sequence identity with the amino acid sequence represented by SEQ ID NO: 7, where a protein including the amino acid sequence is capable of conferring resistance to a plant disease caused by at least one race of at least one *Phytophthora sp.*;
(e5) a fusion amino acid sequence obtained by attaching a tag to the N-terminus and/or C-terminus of the amino acid sequence represented by SEQ ID NO: 7; and
(e6) the amino acid sequence with the same function obtained by substituting and/or deleting and/or adding one or more amino acid residues in the amino acid sequence represented by SEQ ID NO: 7.

In a particular embodiment of this invention, in (e2), the polynucleotide having at least 90% identity with the polynucleotide represented by SEQ ID NO: 1 includes at least one of the polynucleotides represented by SEQ ID NOs: 2-6.

In a particular embodiment of this invention, the polynucleotide having at least 90% identity with the polynucleotide represented by SEQ ID NO: 13 includes at least one of the polynucleotides represented by SEQ ID NOs: 14-18.

In particular embodiments of this invention, the amino acid sequence having at least 90% sequence identity with the amino acid sequence represented by SEQ ID NO: 7 includes at least one of the polynucleotides represented by SEQ ID NOs: 8-12.

In some embodiments, the natural amino acid sequence of the protein may be a variant sequence derived from different species and/or different plants of *Solanum*. The artificial variant amino acid sequence of the above protein may be a variant sequence obtained by appropriately modifying the natural amino acid sequence. The modification includes, but is not limited to: appropriate amino acid substitution/addition/deletion without affecting the biological activity of the target protein, truncation of the N-terminal amino acid, codon optimization suitable for host cell preferences, tag addition, fusion, etc.

Preferably, the amino acid sequence of Rpi-caj1 in this invention is at least one selected from the group consisting of: the amino acid sequences shown in (e1)-(e3), and the artificial variant sequences thereof.

Compared with the prior art, the beneficial effects of this invention are as follows:
The Rpi-caj1 gene of this invention can confer broad-spectrum resistance to late blight infection caused by *Phytophthora infestans* in potatoes, for example, it can simultaneously produce significant resistance to sixteen different strains of *Phytophthora infestans*.

This invention demonstrates that transient expression of the Rpi-caj1 gene in tobacco leaves of *Nicotiana benthamiana* can confer it resistance to late blight.

This invention provides important genetic resources for potato breeding for resistance to late blight, overcomes the problem of loss of disease resistance in potato varieties, and provides new guarantees for the long-term disease resistance of potatoes.

### BRIEF DESCRIPTION OF THE DRAWINGS

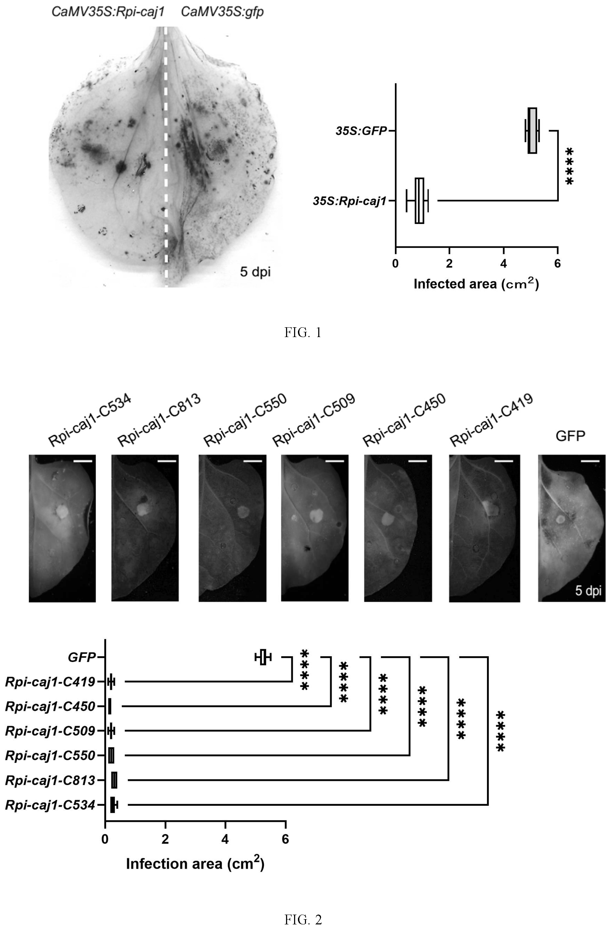

Fig. 1 shows inoculation effect of transient expression of Rpi-caj1-C534 in leaves of *Nicotiana benthamiana* on *Phytophthora* causing late blight. The left side of the leaf transiently expresses Rpi-caj1-C534 (*CaMV35S:Rpi-caj1* construct), and the right side of the leaf is the control (*CaMV35S:gfp*). The blue area in the color image shows the lesions (the black area in the grey image shows the lesions), dpi is an abbreviation for days post inoculation, and 5 dpi means 5 days after inoculation. **** indicates a P value less than 0.0001.
Fig. 2 shows inoculation effect of transient expression of Rpi-caj1 homologous genes from different sources in tobacco leaves on *Phytophthora* causing late blight. **** indicates a P value less than 0.0001.

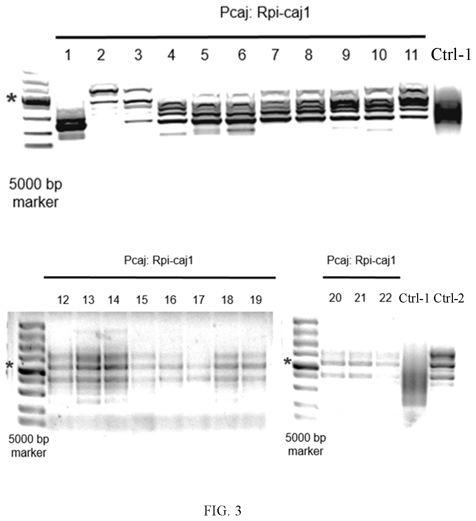

Fig. 3 is electrophoresis diagram of Rpi-caj1-C534 transferred into potato genome. Numbers 1-22 indicate the numbers of the plant lines in which Rpi-caj1-C534 are successfully transferred into potato genome. Control 1 (negative control) is the result of genome amplification of untransformed potato plants, and Control 2 (positive control) is the result of genome amplification of *Solanum cajamarquense* material naturally including the Rpi-caj1-C534 gene. * indicates the position of the target amplification band.

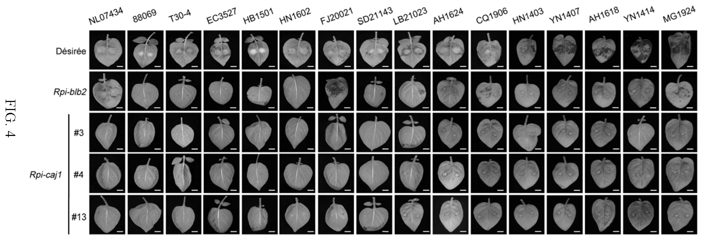

Fig. 4 shows the inoculation effect of Rpi-caj1-C534 transgenic potato leaves on *Phytophthora* causing late blight. Lines #3, #4, and #13 are positive transformant potato lines transfected with the Rpi-caj1-C534 gene, and they are obtained by PCR verification as shown in Fig. 3. They are three independent lines obtained after transformation with the same gene, and are used to verify Rpi-caj1-C534-mediated resistance to late blight.

The English notes in the image are as follows:
Infection area: the area of infection.

Rpi-blb2: Désirée, a potato variety that has been transformed with the Rpi-blb2 gene.

### DETAILED DESCRIPTION

This invention discloses a late blight resistance gene, biomaterial, and use thereof. Those skilled in the art can refer to the content of this invention and appropriately improve the process parameters to achieve the desired outcome. It should be particularly noted that, all such substitutions and modifications are obvious to those skilled in the art, and are considered to be included in this invention. The method and use of this invention have been described through preferred embodiments. Those skilled in the art will be able to modify or appropriately change and combine the method and use described herein without departing from the content, spirit and scope of this invention, so as to implement and apply the technology of this invention.

### Terminology Explanations:

As used herein, the term "nucleic acid molecule" (or "nucleic acid" or "polynucleotide") may refer to a polymeric form of nucleotides, which may include sense and antisense strands of RNA, cDNA, genomic DNA, as well as synthetic forms and mixed polymers described above. Nucleotides can refer to ribonucleotides, deoxyribonucleotides, or any modified form of nucleotides. The term "nucleic acid molecule" as used herein is synonymous with "nucleic acid" and "polynucleotide". The length of a nucleic acid molecule is typically at least 10 bases, unless otherwise stated. This term can refer to RNA or DNA molecules of indeterminate length. This term includes both single-stranded and double-stranded forms of DNA. Nucleic acid molecules may include one or both of naturally occurring and modified nucleotides linked together by naturally occurring and/or non-naturally occurring nucleotides. This invention provides a nucleic acid molecule including the polynucleotide of the Rpi-caj1 gene (especially Rpi-caj1-C534, Rpi-caj1-C813, Rpi-caj1-C550, Rpi-caj1-C509, Rpi-caj1-C450, Rpi-caj1-C419 and an allele, a homolog, an ortholog and other naturally occurring variants of such *R* gene, as well as the synthetic or artificial (i.e. non-natural) variants thereof). Therefore, the genes and polynucleotides in this invention include naturally occurring sequences as well as mutants and other variant forms.

The term "variant" is intended to refer to sequences that are fundamentally similar. For a polynucleotide, a variant include a polynucleotide with deletions (i.e., truncation) at the 5' and/or 3' ends; and/or deletions and/or additions of one or more nucleotides at one or more internal sites in the native polynucleotide; and/or substitutions of one or more nucleotides at one or more sites in the natural polynucleotide. As used herein, a "natural" polynucleotide or polypeptide include naturally occurring nucleotide or amino acid sequences, respectively. For a polynucleotide, a conserved variant include those sequences that encode the amino acid sequence of one of the R proteins of this invention due to the degeneracy of the genetic code. Naturally occurring allele variants may be identified by well-known molecular biology techniques, such as polymerase chain reaction (PCR) and hybridization techniques. Variant polynucleotides also include synthetically derived polynucleotides, such as those produced by site-directed mutagenesis that still encode the R protein of this invention. Typically, as determined by sequence alignment procedures and parameters, a variant of a particular polynucleotide in this invention will have at least about 75%, 80%, 85%, 90%, 91%, 92%, 93%, 94%, 95%, 96%, 97%, 98%, 99% or more sequence identity with that particular polynucleotide. In some embodiments of this invention, a variant of a particular polynucleotide of this invention will have at least about 75%, 80%, 85%, 90%, 91%, 92%, 93%, 94%, 95%, 96%, 97%, 98%, 99% or greater sequence identity with at least one polynucleotide selected from SEQ ID NOs: 1-6 and optionally includes a non-naturally occurring polynucleotide, which differs from the polynucleotides represented by SEQ ID NOs: 1-6 by at least one nucleotide modification selected from the group consisting of: substitution of at least one nucleotide, addition of at least one nucleotide, and deletion of at least one nucleotide. It should be understood that, the addition of at least one nucleotide may be the addition of one or more nucleotides within the polynucleotide of this invention, the addition of one or more nucleotides to the 5' end of the polynucleotide of this invention, and/or the addition of one or more nucleotides to the 3' end of the polynucleotide of this invention.

As will be readily understood by those skilled in the art, a nucleic acid molecule may be chemically or biochemically modified, or can include non-natural or derived nucleotide bases. Such modifications include, for example, labeling, methylation, substitution of one or more naturally occurring nucleotides with analogs, internucleotide modifications (e.g., uncharged bonds: such as methyl phosphonate, triphosphate, phosphoramide, carbamate, etc.; charged bonds: such as thiophosphate, dithiophosphate, etc.; dangling portions: such as peptides; intercalating agents: such as acridine, psoralen, etc.; chelating agents; alkylating agents; and modified bonds: such as α-anomeric nucleic acids, etc.). The term "nucleic acid molecule" also includes any topological conformation, including single-stranded, double-stranded, partially double-stranded, triple-stranded, hairpin, cyclic, and padlock conformations.

As will be readily understood by those skilled in the art, naturally occurring allele variants may be identified by hybridization under strict conditions, where "strict conditions" may be any one of low stringency, medium stringency, or high stringency conditions. "Low stringency conditions", for example, 5×SSC, 5×Denhardt solution, 0.5% SDS, 50% formamide, at 32°C. "Medium stringency conditions", for example, 5×SSC, 5×Denhardt solution, 0.5% SDS, 50% formamide, at 42°C. "High stringency conditions", for example, 5×SSC, 5×Denhardt solution, 0.5% SDS, 50% formamide, at 50°C. Under the above conditions, the higher the temperature, the more efficiently we can expect to obtain DNA with high homology. Factors affecting the stringency of hybridization include temperature, probe concentration, probe length, ionic strength, elongation time, salt concentration, and others. Those skilled in the art can achieve the same stringency conditions by appropriately selecting these factors.

As used herein, the term "identity" refers to sequence similarity to an exemplary nucleic acid sequence or amino acid sequence. Identity may be evaluated by the naked eye or by computer software. By computer software, the identity between two or more sequences may be expressed as a percentage (%), which may be used to evaluate the identity between related sequences.

The identity value of a sequence provided herein refers to an evaluation of the full-length sequence identity performed on a sequence by Dispatcher tools framework (https://www.ebi.ac.uk/services). The identity value of a sequence can also be obtained by using other software, such as a value obtained by using the default parameters in Jalview version 2.11.2.7 (Waterhouse, A.M., Procter, J.B., Martin, D.M.A., Clamp, M. and Barton, G.J. (2009) "Jalview Version 2 - a multiple sequence alignment editor and analysis workbench" Bioinformatics 25 (9) 1189-1191 doi:10.1093/bioinformatics/btp033) with the multiple alignment software MUSCLE version 3.8.31 ("MUSCLE: multiple sequence alignment with high accuracy and high throughput" Nucleic Acids Res.32(5):1792(2004)); or any equivalent program thereof.

Other mathematical algorithms are known in the field, and may be used to compare two sequences. See, for example, the algorithms in Karlin and Altschul (1990) Proc. Natl. Acad. Sci. USA 87:2264, such as Karlin and Altschul (1993) Proc. Natl. Acad. Sci. Modified in USA 90:5873-5877. This algorithm was introduced by Altschul etal. (1990) J. Mol. into the BLAST program of Biol.215:403. A BLAST nucleotide search may be performed by BLASTN program (nucleotide query for polynucleotide search) to obtain polynucleotides homologous to the nucleic acid molecule of this invention, or a BLASTX program (translation nucleotide query for protein sequence search) may be used to obtain protein sequences homologous to the nucleic acid molecule of this invention. A BLAST protein search may be performed by BLASTP program (a protein query for searching protein sequences) to obtain amino acid sequences homologous to the protein molecule of this invention, or a TBLASTN program (a protein query for searching polynucleotides for translation) may be used to obtain polynucleotides homologous to the protein molecule of this invention. To obtain gap alignments for comparative purposes, Gapped BLAST (in BLAST 2.0) may be performed as described by Altschul et al. Nucleic Acids Res. 25:3389 (1997). Alternatively, PSI-Blast may be used to perform iterative searches that detect distant relationships between molecules. See Altschul et al. (1997) supra. When using BLAST, Gapped BLAST, and PSI-Blast programs, you can use the default parameters of the corresponding programs (such as BLASTX and BLASTN). The comparison can also be performed manually through inspection.

As used herein, the term "homology" is sometimes used to refer to the level of similarity (i.e., sequence similarity or identity) between two or more nucleic acids or amino acid sequences, expressed as a percentage of positional identity. Homology also refers to the concept of evolutionary correlation, which is usually demonstrated by the similar functional properties between different nucleic acids or proteins that share similar sequences.

In some embodiments, the homology sequences related to this invention may be obtained by comparing exemplary sequences in sample genomic or transcriptomic data of evolutionarily closely related species. For example, between different species of the same genus, or between different plants of the same species, homologous sequences obtained by comparing exemplary sequences in sample genomic or transcriptomic data may be expected by those skilled in the art to have the same or similar functions.

As used herein, the term "CDS sequence" refers to a coding sequence. DNA is transcribed into mRNA, and mRNA is then processed through splicing and other processes to translate into proteins. A CDS is a DNA sequence that corresponds one-to-one with a protein sequence, and this sequence does not include any other sequences that do not correspond to the protein. It completely corresponds to the protein's codons, regardless of sequence changes during mRNA processing.

In some embodiments, the nucleic acid molecules associated with this invention may be cloned from DNA including a given nucleic acid molecule from any source, for example by PCR amplification and/or restriction enzyme digestion. In some embodiments, the nucleic acid molecules associated with this invention are synthetic. Any method for obtaining the nucleic acid molecules related to the invention is compatible with this invention.

As used herein, the term "synthetic" refers to polynucleotide (i.e., DNA or RNA) molecules produced by chemical synthesis as an in vitro process. For example, synthetic DNA may be generated during the reaction process within the Eppendorf^{™} tube, allowing the synthetic DNA to be enzymatically produced from natural DNA or RNA chains. Other laboratory methods may be used to synthesize polypolynucleotides. Oligonucleotides may be chemically synthesized on an oligonucleotide synthesizer by solid-phase synthesis with phosphoramide. Synthetic oligonucleotides can anneal each other as complexes to produce "synthetic" polynucleotides. Other methods for the chemical synthesis of polynucleotides are known in the art, and may be readily implemented for use in this invention.

As used herein, the term "gene" refers to a fragment of nucleic acid that expresses a specific protein. A "gene" includes the DNA region that encodes the gene product, as well as all DNA regions that regulate the generation of the gene product, regardless of whether such regulatory sequences are adjacent to coding and/or transcriptional sequences. Therefore, genes include, but are not limited to: promoter sequence, terminator, translation regulatory sequence such as a ribosome binding site and an internal ribosome entry site, enhancer, silencer, insulator, boundary element, origin of replication, matrix attachment site, intron, and locus control region.

As used herein, the term "gene product" means any product produced by a gene. For example, a gene product may be a direct transcription product of the gene (such as mRNA, tRNA, rRNA, antisense RNA, interfering RNA, ribozyme, structural RNA, or any other type of RNA), or it may be a protein produced by translating mRNA.

As used herein, the term "protein" (or "protein" or "peptide" or "polypeptide" or "peptide composition") includes both naturally occurring proteins and variants and modifications thereof. The terms "protein" and "polypeptide" used herein are used interchangeably; therefore, the term polypeptide can refer to a full-length polypeptide or a fragment of a full-length polypeptide. The term "fragment" refers to a portion of a polypeptide sequence. "Fragment" or "biologically active portion" includes polypeptides including a sufficient number of adjacent amino acid residues to retain biological activity, such as peptides with truncated N-terminal amino acids.

"Variant" proteins are proteins derived from natural proteins by the deletion (so-called truncation) of one or more amino acids at the N-terminus and/or C-terminus of the natural protein, the deletion and/or addition of one or more amino acids at one or more internal sites in the natural protein, or the substitution of one or more amino acids at one or more sites in the natural protein. Such variants may be generated, for example, through genetic polymorphism or artificial manipulation. As determined by sequence alignment procedures and parameters, bioactive variants of the R protein will have at least about 75%, 80%, 85%, 90%, 91%, 92%, 93%, 94%, 95%, 96%, 97%, 98%, 99% or greater sequence identity with the amino acid sequences of this invention (e.g., the amino acid sequences represented by SEQ ID NOs: 7-12). The bioactive variants of the protein described in this invention may differ from the protein in having as few as 1-15 amino acid residues, as few as 1-10, such as 6-10, as few as 5, as few as 4, 3, 2 or even 1 amino acid residue. More preferably, such variants confer enhanced resistance to a plant disease caused by at least one race of at least one *Phytophthora sp*. In some embodiments, mutations generated in the DNA encoding the variant will not place the sequence outside the reading frame. Ideally, the mutation should not produce a complementary region that could generate a secondary mRNA structure.

The proteins of this invention may be modified in various ways, including amino acid substitution, deletion, truncation, and insertion. The methods used for such operations are generally known in the art. Methods for mutagenesis and polynucleotide alteration are well known in the art. For guidance on appropriate amino acid substitutions that do not affect the biological activity of the target protein, see the model described in Dayhoff et al. (1978) Atlas of Protein Sequence and Structure (Natl. Biomed. Res. Found., Washington, D.C.), which is incorporated herein in its entirety by reference. Conservative substitutions, such as replacing one amino acid with another amino acid that has similar properties, may be optimal.

It is expected that the deletions, insertions, and substitutions of the protein sequences included in this invention will not produce fundamental changes in the protein characteristics. However, when it is difficult to predict the exact effect of substitution, deletion or insertion, those skilled in the art will understand that the effect will be evaluated by conventional screening assays. In other words, activity may be assessed by the assays disclosed below.

Variant polynucleotides and proteins also include sequences and proteins derived from mutagenesis and recombination processes, such as DNA shuffling. This DNA shuffling strategy is known in the field.

As used herein, the term "amino acid" means any amino acid (both standard and non-standard amino acids), including but not limited to: α-amino acid, β-amino acid, γ-amino acid and δ-amino acid. Examples of suitable amino acids include, but are not limited to: alanine, asparagine, aspartic acid, cysteine, glutamic acid, glutamine, glycine, proline, serine, tyrosine, arginine, histidine, isoleucine, leucine, lysine, methionine, phenylalanine, threonine, tryptophan, and valine.

As used herein, the term "isolation/isolating" means removal from its natural environment or from other compounds present when the compound was first formed. The term "isolated" includes materials isolated from natural sources as well as materials (such as nucleic acids and proteins) that are prepared and then recovered through recombinant expression in host cells, or chemically synthesized compounds such as nucleic acid molecule, protein, and peptide. Any method for obtaining the proteins relevant to the invention is compatible with this invention.

As used herein, the term "expression cassette" refers to a DNA fragment that may be inserted into a nucleic acid or polynucleotide at a specific restriction site or through homologous recombination. As used herein, the DNA fragment includes a polynucleotide encoding a target polypeptide, and the expression cassette and restriction sites are designed to ensure that the expression cassette is inserted into the appropriate reading frame for transcription and translation. In one embodiment, the expression cassette may include a polynucleotide encoding a target polypeptide and, in addition to the polynucleotide, an element that promotes transformation of a specific host cell. In one embodiment, the expression cassette may further include an element that allows for enhanced expression of the polynucleotide encoding the target polypeptide in the host cell. These elements may include, but are not limited to: promoter, minimal promoter, enhancer, responsive element, terminator sequence, polyadenylation sequence, etc.

The expression cassette may also include a selection marker gene for selecting the transformed cells. Selective marker genes are used to select cells or tissues for transformation. Marker genes include genes encoding antibiotic resistance, such as genes encoding neomycin phosphotransferase II (NEO) and hygromycin phosphotransferase (HPT), as well as genes conferring resistance to herbicides such as glufosinate, bromobenzonitrile, imidazolinone and 2,4-dichlorophenoxyacetate (2,4-d)). Other selection markers include phenotypic markers, for example β-galactosidase, and fluorescent proteins such as green fluorescent protein (GFP), cyan fluorescent protein (CYP), and yellow fluorescent protein.

As used herein, the term "vector" is used interchangeably with "construct," "cloning vector," and "expression vector," and means a vector that can introduce a DNA or RNA sequence (e.g., an exogenous gene) into a host cell to transform the host and promote the expression (e.g., transcription and translation) of the introduced sequence. "Non-viral vector" refers to any vector that does not include virus or retrovirus. In some embodiments, a "vector" is a DNA sequence including at least one DNA replication origin and at least one selective marker gene. Examples include, but are not limited to: a plasmid, cosmid, phage, bacterial artificial chromosome (BAC), or virus that carries a foreign DNA into a cell. The vector may also include one or more genes, antisense molecules and/or selective marker genes and other genetic elements known in the art. The vector can transduce, transform or infect cells, thereby causing the cells to express nucleic acid molecules and/or proteins encoded by the vector. The term "plasmid" refers to a circular chain of nucleic acid that can replicate on an autosome in prokaryotic or eukaryotic host cells. The term includes nucleic acids, which may be DNA or RNA, and may be single-stranded or double-stranded. The plasmid defined may also include sequences corresponding to bacterial replication origins.

In some embodiments, the "cloning vector" can autonomously replicate or integrate into the host cell genome. It is also characterized by one or more restriction endonuclease sites, at which the vector may be cleaved in a defined manner and the desired DNA sequence may be ligated into the vector, so that the new recombinant plasmid retains its ability to replicate in the host cell. In the case of plasmids, the replication of the desired sequence may occur multiple times as the number of copies of the plasmid in the host cell (such as a bacterial host) increases, or it may occur only once in each host before the host reproduces by mitosis. In the case of bacteriophages, replication can occur actively during the lysis phase, or passively during the lysogenic phase.

In some embodiments, the "expression vector" may be inserted with a desired DNA sequence through restriction enzyme digestion and ligation, so that it is effectively linked to the regulatory sequence and may be expressed as an RNA transcript. The vector may also include one or more marker sequences suitable for identifying whether cells have been transformed or transfected by the vector. A marker includes a gene encoding a protein that increases or decreases resistance or sensitivity to antibiotics or other compounds, a gene encoding an enzyme whose activity may be detected by standard methods known in the art (e.g., β-galactosidase, luciferase, or alkaline phosphatase), and a gene having a visible effect on the phenotype of transformed or transfected cells, hosts, colonies, or plaques (e.g., green fluorescent protein). Preferred vectors are those that can autonomously replicate and express the products of structural genes present in the DNA fragments that are effectively linked to them.

As used herein, the term "expression" refers to the biosynthesis of a gene product, including the transcription and/or translation of the gene product. "Expressing" or "producing" a protein or polypeptide from a DNA molecule refers to transcribing and translating the coding sequence to produce the protein or polypeptide, while "expressing" or "producing" a protein or polypeptide from an RNA molecule refers to translating the RNA coding sequence to produce the protein or polypeptide.

Gene expression may be affected by external signals, such as when a cell, tissue, or organism is exposed to a substance that can increase or decrease gene expression. Gene expression may be regulated anywhere along the process from DNA to RNA to protein. Gene expression may be regulated by controlling transcription, translation, RNA transport and processing, degradation of intermediate molecules (such as mRNA), or by the activation, inactivation, segmentation or degradation of specific protein molecules after their generation, or by a combination of these. The exact nature of the regulatory sequences required for gene expression may vary between species or cell types, but generally should include, as needed, 5' non-transcriptional and 5' non-translational sequences involved in transcription and translation initiation, such as TATA box, capped sequence, CAAT sequence, etc. In particular, this 5' non-transcriptional regulatory sequence will include a promoter region, which includes a promoter sequence that controls transcriptional control of a gene operatively linked. The regulatory sequence may also include an enhancer sequence or a desired upstream activator sequence. The vector of this invention may optionally include a 5' preamble or signal sequence. The selection and design of a suitable vector is within the capabilities and judgment of a person skilled in the art.

In some embodiments, when a nucleic acid molecule encoding any enzyme of this invention is expressed in a cell, a variety of transcriptional control sequences (e.g., promoter/enhancer sequences) may be used to guide its expression. A promoter may be a natural promoter, that is, in its endogenous environment, a gene promoter provides normal regulation of gene expression. In some embodiments, the promoter may be constitutive, that is, the promoter continuously transcribes its associated gene without regulation. Various conditional promoters can also be used, such as promoters controlled by the presence or absence of a molecule. Chemically regulated promoters may be used to regulate gene expression in the host by applying exogenous chemical regulators. Depending on the purpose, a promoter may be a chemically induced promoter, in which the invention of a chemical substance induces gene expression, or a chemically repressive promoter, in which the invention of a chemical substance represses gene expression. Chemically induced promoters are known in the art, including but not limited to: the corn In2-2 promoter (which is activated by benzenesulfonamide herbicide safener), the corn GST promoter (which is activated by a hydrophobic electrophilic compound used as a pre-germination herbicide), and the tobacco PR-1a promoter (which is activated by salicylic acid). Other promoters of interest that regulate chemical substances include glucocorticoid-inducible promoters in steroid-responsive promoters, as well as tetracycline-inducible and tetracycline-repressive promoters.

The expression vector including all the necessary expression elements is commercially available, and is well known to those skilled in the art. For example, see Sambrook et al., Molecular Cloning: A Laboratory Manual, 2nd ed., Cold Spring Harbor Laboratory Press, 1989. Genetic engineering of cells involves introducing exogenous DNA (RNA) into them. The exogenous DNA (RNA) is placed under the effective control of transcriptional elements to allow the exogenous DNA to be expressed in the host cell.

As used herein, the term "transformation" includes all techniques that can introduce nucleic acid molecules into such cells. Examples include, but are not limited to: transfection with viral vectors; plasmid vector transformation; electroporation; fat infection; microinjection; *Agrobacterium*-mediated transfer; direct DNA uptake; Whiskers^{™} mediated transformation; and micro-projectile bombardment. These technologies may be used for both stable and transient transformation of host cells. "Stable transformation" refers to the introduction of nucleic acid fragments into the genome of a host organism, resulting in genetic stability. Once the transformation is stable, the nucleic acid fragments are stably integrated into the host organism and the genome of any subsequent generations. Host organisms including transformed nucleic acid fragments are called "genetically modified" organisms. "Transient transformation" refers to the introduction of nucleic acid fragments into the cell nucleus or DNA-including organelles of a host organism, resulting in gene expression but failing to achieve stable heritability.

In some embodiments, in order to transform the host and host cells, the polynucleotide of this invention may be inserted into any vector known in the art that is suitable for expressing the polynucleotide in the host or host cells by standard techniques. The choice of vector depends on the preferred transformation technology and the target host species to be transformed. The transformation method depends on the host cell to be transformed, the stability of the vector used, the expression level of the gene product, and other parameters.

As used herein, the term "plant" includes seeds, plant cells, plant protoplasts, plant cell tissue cultures from which plants can regenerate, plant callus, plant masses, and complete plant cells in plants or plant parts such as embryos, pollen, ovules, seeds, tubers, propagules, leaves, flowers, branches, fruits, roots, root tips, anthers, etc. The offspring, variants, and mutants of regenerated plants are also included within the scope of this invention, provided that these portions include introduced polynucleotides. As used herein, unless otherwise explicitly stated or obvious from the context of use, "offspring" and "offspring plant" include any subsequent generations of a plant, whether produced by sexual and/or asexual reproduction.

The terms "transgenic plant" and "transformed plant" are equivalent terms to "plant" as described above, where a plant includes a heterologous nucleic acid molecule, heterologous polynucleotide, or heterologous polynucleotide construct introduced into the plant by any stable and transient transformation method disclosed elsewhere herein or otherwise known in the art. Such transgenic and transformed plants also refer to, for example, plants in which a heterologous nucleic acid molecule, heterologous polynucleotide, or heterologous polynucleotide construct is first introduced, as well as any one of their offspring plants including said heterologous nucleic acid molecule, heterologous polynucleotide, or heterologous polynucleotide construct.

In some embodiments, the methods of this invention are used to produce plants including heterologous polynucleotides, where the heterologous polynucleotides include the *R* gene polynucleotide of this invention, and the methods involve genome editing to modify the polynucleotide of a natural or non-natural gene in the genome of the plant. The natural or non-natural genes described herein include polynucleotides that are different from the *R* gene polynucleotide of this invention, and the modified natural or non-natural genes include the *R* gene polynucleotide of this invention. Typically, such methods involve using a plant whose genome includes a natural or non-natural gene, where the natural or non-natural gene includes a polynucleotide homologous to the *R* gene polynucleotide of this invention, and the methods also involve introducing a nucleic acid molecule including at least a portion of the *R* gene polynucleotide of this invention into the plant. Preferably, the polynucleotide of the natural or non-natural gene has about 70%, 75%, 80%, 85%, 90%, 95%, 96%, 97%, 98%, 99% or higher polynucleotide identity with at least one *R* gene polynucleotide of the present invention. Such natural or non-natural genes may be, for example, non-functional homologs of the *R* gene, particularly the *Rpi-caj1* gene, that cannot or are unknown whether they can confer resistance to a plant disease.

The term "solanaceous plant" is intended to include any solanaceous plant at any stage of maturity or development, and unless otherwise explicitly stated in the context, any cell, tissue, or organ (plant part) obtained from or derived from any such plant. Solanaceous plants include, but are not limited to: fruits, stems, tubers, roots, flowers, ovules, stamens, leaves, embryos, meristematic regions, callus, anther cultures, gametophytes, sporophytes, pollen, microspores, protoplasts, etc. As used herein, the term "tuber" is intended to refer to the whole tuber or any part thereof, such as a slice or portion of a potato tuber including one or more buds (i.e., "eyes") suitable for field cultivation to produce potato plants. This invention also includes seeds produced from the solanaceous plants described herein.

In some embodiments of this invention, the plant of this invention, particularly a solanaceous plant, may include one, two, three, four, five, six or more polynucleotides encoding R proteins. Normally, but not necessarily, two or more R proteins are different from each other. In this invention, when two R proteins have different amino acid sequences, the R protein is different from the other R protein. In some embodiments of this invention, each of the different R proteins used to resist a plant disease caused by a race of *Phytophthora sp*. has one or more differences in resistance characteristics, such as resistance to different races and/or groups of races of the same *Phytophthora* species or even different species of the same *Phytophthora*. By combining two, three, four, five, six or more polynucleotides with each of those encoding different R proteins for resistance to different races of a species of the *Phytophthora* or certain species of the *Phytophthora*, solanaceous plants with broad-spectrum resistance to multiple races of a single species of *Phytophthora* or even multiple species of *Phytophthora* may be produced. This type of solanaceous plants, especially potato or tomato plants, may be used in agriculture in areas where multiple races of a particular species of *Phytophthora* (e.g., multiple races of pathogenic *Phytophthora*) are prevalent.

The plant of this invention including multiple *R* genes may be produced, for example, by transforming a plant that already includes one or more other *R* gene polynucleotides with a heterologous polynucleotide including at least one Rpi-caj1 polynucleotide of this invention. Such a plant, which already includes one or more other *R* gene polynucleotides, may include: the *R* gene naturally occurring in the genome or the plant, the *R* gene introduced into the plant through sexual reproduction, or the *R* gene introduced into the plant or its ancestor through transformation with the *R* gene polynucleotide. Alternatively, one or more other *R* gene polynucleotides may be introduced into the plant of this invention by, for example, transformation or sexual reproduction, where the plant already includes the heterologous polynucleotide of this invention.

In other embodiments, two or more different *R* gene sequences may be introduced into a plant by stably transforming the plant with a heterologous polynucleotide or vector including two or more *R* gene polynucleotides. Alternatively, the heterologous polynucleotides of this invention may be integrated into the genome of a plant, adjacent to another *R* gene polynucleotide, by genome modification methods based on homologous recombination described elsewhere herein or known in the art.

As used herein, the terms "DNA" or "RNA" is not intended to limit this invention to polynucleotide molecules including DNA or RNA. Those skilled in the art will recognize that, the method and composition of this invention include polynucleotide molecules consisting of deoxyribonucleotides (i.e., DNA), ribonucleotides (i.e., RNA), or combinations of ribonucleotides and deoxyribonucleotides. Such deoxyribonucleotides and ribonucleotides include naturally occurring molecules and synthetic analogs, including but not limited to nucleotide analogs or modified backbone residues or linkages, which are synthetic, naturally occurring or non-natural, have binding properties similar to reference nucleic acids, and are metabolized in a manner similar to reference nucleotides. Examples of such analogues include, but are not limited to: thiophosphate, aminophosphate, methylphosphonate, chiral methylphosphonate, 2-O-methylribonucleotide, and peptide-nucleic acid (PNA). The polynucleotide molecules in this invention also include all forms of polynucleotide molecules, including but not limited to: single-stranded, double-stranded, hairpin, stem-loop, etc. Furthermore, those skilled in the art will understand that, the polynucleotides disclosed herein also include complementary sequences of the exemplary polynucleotides.

**Table 1. Species information of the genes of this invention.**

| Gene names | Species of origin | Similarity of nucleic acid with Rpi-caj1-C534 (%) | Similarity of amino acids with Rpi-caj1-C534 (%) |
|---|---|---|---|
| Rpi-caj1-C534 | *Solanum cajamarquense* | 100 | 100 |
| Rpi-caj1-C813 | *Solanum cajamarquense* | 99.73 | 99.67 |
| Rpi-caj1-C550 | *Solanum sogarandinum* | 97.69 | 97.36 |
| Rpi-caj1-C509 | *Solanum cardiophyllum* | 98.13 | 97.69 |
| Rpi-caj1-C450 | *Solanum piurae* | 97.69 | 97.69 |
| Rpi-caj1-C419 | *Solanum candolleanum* | 97.8 | 97.19 |

**Table 2. Sequence information of genes and proteins of this invention.**

| Gene names | Gene sequence No. (SEQ ID NO) | Amino acid sequence No. (SEQ ID NO) | CDS sequence No. (SEQ ID NO) | Promotor sequence No. (SEQ ID NO) |
|---|---|---|---|---|
| Rpi-caj1-C534 | 1 | 7 | 13 | 19 |
| Rpi-caj1-C813 | 2 | 8 | 14 | 20 |
| Rpi-caj1-C550 | 3 | 9 | 15 | 21 |
| Rpi-caj1-C509 | 4 | 10 | 16 | 22 |
| Rpi-caj1-C450 | 5 | 11 | 17 | 23 |
| Rpi-caj1-C419 | 6 | 12 | 18 | 24 |

Unless otherwise specified, all reagents, instruments, strains or biomaterials used in this invention are commercially available. The *Phytophthora infestans* strains JH19, NL07434, 88069, T30-4, EC3527, HB1501, HN1602, FJ20021, SD21143, LB21023, AH1624, CQ1906, HN1403, YN1407, AH1618, YN1414, and MG1924 used in the following examples were all obtained from the Crop *Phytophthora* Genomics Laboratory of the Department of Plant Pathology, College of Plant Protection, Nanjing Agricultural University.

The present invention is further illustrated below with reference to the following Examples:

### Example 1

### 1. Cloning of the Rpi-caj1 Gene

Primers for amplifying the Rpi-caj1 gene were designed based on the polynucleotide of the Rpi-caj1 gene. gDNA from wild potato leaves was used as a template, the Rpi-caj1 gene was amplified by PCR. The PCR products were recovered by gel extraction, and then recombined and linked with the linear pBin307 vector digested with SmaI enzyme. The plasmid was cloned and sequenced by using the Vazyme C115 homologous recombination kit (ClonExpress Ultra One Step Cloning Kit). The positive recombinant plasmid was named pBin307-Rpi-caj1.

The primer sequences are shown in the Table below.

**Table 3: Primer sequences**

| Rpi-caj1 gene names | Origin (Wild potato) | Sequences |
|---|---|---|
| Rpi-caj1-C534 | *Solanum cajarmarquense* C534 | F:5'- TCGACGGTACCGCGGGCCCATGGCAACCCCACAAA CCCGTTATT-3' (SEQ ID NO: 25), |
| | | R:5'- TAGACTAGGTGGATCCCCTACCAAGACAAGCTTAAC ATGAAT-3'(SEQ ID NO: 26) |
| Rpi-caj1-C534* | *Solanum cajarmarquense* C534 | F:5'- GTTAATTAAGAATTCACCGGGGTTGATCTCCTTTACT TGTGCTAC-3'(SEQ ID NO: 27), |
| | | R:5'- TAGACTAGGTGGATCCCTCATGGGAAGAATCTCTTG AGGCTACCAG-3'(SEQ ID NO: 28) |
| Rpi-caj1-C813 | *S. cajamarquense* C813 | F:5'- CGAATTCTGCAGTCGACcccATGGCAACCCCACAAAC CCGTTATTC-3' (SEQ ID NO: 29), |
| | | R:5'- TACAAGAAAGCTGGGTCcccTGGGAAGAATCTCTTGA GGCTACCAG-3' (SEQ ID NO: 30) |
| Rpi-caj1-C550 | *S. sogarandinum* C550 | F:5'- CGAATTCTGCAGTCGACcccATGGCAACCCCACAAAC CCGTTATTC-3' (SEQ ID NO: 31), |
| | | R:5'- TACAAGAAAGCTGGGTCcccTGGGAAGAATCTTTTGA GGCCACCAG-3' (SEQ ID NO: 32) |
| Rpi-caj1-C509 | *S. cardiophyllum* C509 | F:5'- CGAATTCTGCAGTCGACcccCGCAATTGCCTTAACAA ACAAAC-3' (SEQ ID NO: 33), |
| | | R:5'- TACAAGAAAGCTGGGTCcccTGGGAAGAATCTTTTGA GGCCACCAG-3' (SEQ ID NO: 34) |
| Rpi-caj1-C450 | *S. piurae* C450 | F:5'- CGAATTCTGCAGTCGACcccATGGCAACCCCACAAAC CCGTTATTC-3' (SEQ ID NO: 35), |
| | | R:5'- TACAAGAAAGCTGGGTCcccTGGGAAGAATCTTTTGA GGCCACCAG-3' (SEQ ID NO: 36) |
| Rpi-caj1-C419 | *S. candolleanum* C419 | F:5'- CGAATTCTGCAGTCGACcccATGGCAACCCCACAAAC CCG-3' (SEQ ID NO: 37), |
| | | R:5'- TACAAGAAAGCTGGGTCcccTGGGAAGAATCTTTTGA GGCCAC-3' (SEQ ID NO: 38) |

| | | |
|---|---|---|
| Note: * indicates that the corresponding primer pair is used for cloning genes including promoters. | | |

### PCR Reaction System and Conditions:

PCR reaction system: 0.2 µM, 1×PCR buffer, 0.2 mM dNTPs, 3.75 mM MgCl₃, with 2U of DNA polymerase per 25 µL reaction system (TaKaRa Biotechnology, Dalian).

PCR reaction conditions: Step 1, 94 °C, 5 min; Step 2, 94 °C, 1 min; Step 3, 56 °C, 2 min; Step 4, 72 °C, 2 min; repeating Steps 2-4 for 35 times; Step 6, 72 °C, 10 min; Step 7, 4 °C, constant temperature.

### Enzyme Digestion System and Conditions:

Enzyme digestion reaction system: restriction endonuclease, 2 units; plasmid DNA, 1 µg; 10× NE Buffer, 1 µL (1×); total reaction system: 20 µL.

Enzyme digestion reaction conditions: the digestion temperature of SmaI enzyme is 25 °C, and the incubation time is 60 min.

### Recombination System and Conditions:

Recombinant reaction system: linearized plasmid, 1 µL; PCR products of target gene, 2 µL; 2× ClonExpress Mix, 5 µL; total reaction system: 10 µL.

Recombinant reaction conditions: temperature: 50 °C, Time: 5 min.

2. The following method was used to transform *E. coli* with plasmid homologous recombination products:
1). setting the temperature of the constant temperature water bath to 42 °C beforehand;
2). taking a tube (100µL) of DH5α *Escherichia coli* competent cells from a -70 °C ultra-low temperature freezer, immediately thawing it with your finger, then inserting it into the ice to incubate for 5-10 min;
3). adding 5 µL of the recombinant plasmid mixture (DNA content not exceeding 100 ng), gently shaking, and placing on ice for 20 min;
4). after gently shaking, immersing the sample in a 42°C water bath for 1-2 min to induce heat shock, then quickly returning it to the ice and then standing for 3-5 min.
5). on a clean bench, adding 500 µL of LB medium (antibiotic-free) to each of the above tubes, mixing gently, and then fixing them on the spring frame of a shaker to shake at 37°C for 1 h;
6). on a clean bench, taking 100-300 µL of the above transformation mixture to drop it onto solid LB agar plates including 50 µg/mL kanamycin, then spreading the mixture evenly with a glass spreader burned with an alcohol lamp.

3. Plasmid Extraction was Performed by Vazyme Plasmid Mini Kit (FastPure Plasmid Mini Kit).

4. Transformation of Competent *Agrobacterium* Cells with Plasmids:
1). adding 5-10 µL of the prepared plasmid DNA to 100 µL of *Agrobacterium* GV3101 competent cells;
2). incubating the *Agrobacterium*-plasmid mixture in a 37°C water bath for 5 min;
3). adding 1 ml of LB liquid medium to the *Agrobacterium*-plasmid mixture to incubate with shaking at 28°C for 2-4 h;
4). centrifuging the *Agrobacterium* (10000g, 30s), removing 0.9mL of supernatant, and resuspending the *Agrobacterium* cells in the remaining supernatant;
5). spreading the suspension evenly on LB solid medium plates including 50 µg/mL kanamycin to incubate at 28°C for 2 days.

**Example 2: Transient Expression of Rpi-caj1-C534 in Tobacco Leaves of *Nicotiana benthamiana* and Inoculation with *Phytophthora infestans*.**

100 µL of the *Agrobacterium* tumefaciens culture prepared in Example 1 at -80 °C was taken to put into a 1.5 mL centrifuge tube, then adding 1 mL of LB (Kana+Rif) liquid medium to incubate at 28 °C, 220 rpm for 12 h on a shaker. Then 100 µL of the culture was taken to put into a 50 mL centrifuge tube, adding 10 mL of LB (Kana+Rif) liquid medium to incubate at 28 °C, 220 rpm on a shaker overnight. The OD value of the culture was detected, and the required OD₆₀₀ should be approximately 0.5.

*Agrobacterium* strains including Rpi-caj1 binary expression vector pBin307-Rpi-caj1 were collected by centrifugation, then resuspending in MES buffer, and OD₆₀₀ = 0.3 were adjusted. The *Agrobacterium* strains were injected into one side (left side) of 4-week-old leaves of wild-type tobacco plants of *Nicotiana benthamiana*. *Agrobacterium* strains including pBin307-gfp were injected into the other side (right side) of the leaves as a negative control. One day after injection, 10 µL of zoospore droplets of *Phytophthora infestans* strain JH19, which includes 200,000 to 400,000 spores per milliliter, were inoculated onto both sides of the leaves. Results were collected 5 days after inoculation (5 dpi). Resistance is evaluated directly by observing lesions on the leaves; if the plant is resistant, no lesions will appear.

The results showed that under the CaMV35S promoter, Rpi-caj1 could produce punctate weak necrosis on tobacco leaves of *Nicotiana benthamiana*, but the large-area necrosis caused by zoospore infection of *Phytophthora infestans* was significantly reduced on the leaf side transiently expressing Rpi-caj1. This indicates that transient expression of Rpi-caj 1 in tobacco leaves of *Nicotiana benthamiana* can produce significant resistance to inoculation with zoospores of *Phytophthora infestans* (Fig. 1).

**Example 3: Transient Expression of Rpi-caj1 Homologous Genes from Different Sources in Tobacco Leaves and Inoculation with *Phytophthora infestans.***

The method is the same as in Example 2.

Compared with the GFP control, leaves transiently expressing Rpi-caj1-C534, Rpi-caj1-C813, Rpi-caj1-C550, Rpi-caj1-C509, Rpi-caj1-C450, and Rpi-caj1-C419 showed significant resistance to *Phytophthora infestans.* The results showed that the Rpi-caj1 homologous genes from different sources could mediate resistance to *Phytophthora infestans* (Fig. 2).

### Example 4: Transfection of Rpi-caj1-C534 into Potato Genome

In this example, the expression of the disease resistance gene Rpi-caj 1 is driven by the promoter Pcaj of the disease resistance gene itself derived from wild potato, and the expression of the nptII gene is driven by the CamV 35S promoter. They are constructed in the plant expression vector pBin307 (same as in Example 1). By using the stems of the potato susceptible varieties Désirée obtained by *Agrobacterium-mediated* stable transformation, transgenic plants were finally obtained. Through kanamycin resistance screening and phenotypic verification, transgenic materials resistant to late blight and tolerant to kanamycin herbicide were obtained. Particularly, the method of genetic modification is as follows:

### (1) Stem Segment Pre-culture:

Z1N2 medium, with two sheets of sterile filter paper on top, 2 mL of PACM (plant MS liquid medium including 1 µg/mL 2,4-dichlorophenoxyacetic acid and 0.5 µg/mL kinetin) was added thereto, a certain number of stem segments were cut to arrange them neatly, then incubating under light (48 h).

### (2) Activation of Microbial Strains:

100 µL *of Agrobacterium* liquid culture at -80 °C was taken to place in a 1.5 mL centrifuge tube, then adding 1 mL of LB (Kana+Rif) liquid medium to incubate at 28 °C, 220 rpm for 12 h on a shaker. Then 100 µL of *Agrobacterium* liquid culture was taken to place in a 50 mL centrifuge tube, adding 10 mL of LB (Kana+Rif) liquid medium to incubate at 28°C, 220 rpm on a shaker overnight. The OD value was detected, and the required OD₆₀₀ should be approximately 0.5.

### (3) Infection:

The *Agrobacterium* liquid culture was centrifuged (4 °C, 4000r, 10min) to collect the bacterial pellet, then resuspending in MS20 liquid medium (adding 1‰ acetyleugenone AS, adding 10µL AS per 10mL of MS20 liquid medium) to the OD₆₀₀ value of 0.5. The pre-cultured stem segments were placed in the resuspended bacterial solution for 10 min, shaking continuously during this time. Stem segments were collected and cultured in Z1N2 AS medium (with filter paper) at 24°C in the dark for 48 hours.

### (4) Differentiation:

Stem segments were cultured in differentiation medium Z2N0.01. The culture medium was changed every two weeks. When the callus tissue differentiates into shoots, transferring it to rooting medium (one liter of MS30 + 2 mL 300 mg/mL Timentin (TMT) + 1 mL 50 mg/mL kanamycin), subculturing every 3-4 weeks.

### (5) Screening for Transgenic Positive Plants

Once the potato seedlings on the rooting medium have rooted and grown to a certain size, they may be screened. Leaves of the plant were taken, and DNA was extracted for PCR verification.

Primers were designed based on the Rpi-caj1 gene sequence. PCR amplification was performed by using genomic DNA from transgenic potato leaves as a template. The reaction conditions were: 95 °C for 5 min; 95 °C for 30 sec, 58 °C for 30 sec, 72 °C for 2 min, Go to 2-30 cycles; 72 °C for 8 min. The PCR product is approximately 350 bp in size. The results showed that the target gene Rpi-caj1 was integrated into the potato genome (Fig. 3).

### Example 5: Inoculation of Transgenic Potato Leaves with Phytophthora infestans.

In this example, the late blight resistance identification of transgenic potatoes was performed by using zoospores of the *Phytophthora infestans* strains collected from petri dishes. 10 µL of *Phytophthora infestans* zoospore droplets including 200,000 to 400,000 spores per mL were inoculated onto detached leaves of potato plants obtained by the method of Example 4 at 4-6 week-age. The results were recorded on day 5 after inoculation. Eight separate leaves were used in each inoculation experiment, and each experiment was repeated at least three times.

The results showed that, compared with untransformed potatoes (Désirée), the transgenic potato plants stably expressing Rpi-caj1-C534 exhibited significant resistance to 16 different *Phytophthora infestans* strains (NL07434, 88069, T30-4, EC3527, HB1501, HN1602, FJ20021, SD21143, LB21023, AH1624, CQ1906, HN1403, YN1407, AH1618, YN1414, and MG1924). Their resistance spectrum was superior to the known resistance gene Rpi-blb2, demonstrating that Rpi-caj1-C534 can mediate broad-spectrum resistance of potatoes to late blight (Fig. 4).

The above description is only preferred embodiments of this invention. It should be noted that for those skilled in the art, several improvements and modifications may be made without departing from the principle of this invention, and these improvements and modifications should also be considered within the protection scope of this invention.

## Claims

1. A nucleic acid molecule, **characterised by** comprising at least one polynucleotide selected from the group consisting of:
(a1) the polynucleotide represented by SEQ ID NO: 1;
(a2) a polynucleotide encoding the amino acid sequence represented by SEQ ID NO: 7;
(a3) the polynucleotide represented by SEQ ID NO: 13;
(a4) a polynucleotide having at least 90% sequence identity with any one of the polynucleotides shown in (a1) and (a3); and
(a5) a polynucleotide encoding any one of the amino acid sequences having at least 90% sequence identity with the amino acid sequence shown in (a2).

2. The nucleic acid molecule according to claim 1, **characterised in that** in (a4), the polynucleotide having at least 90% identity with the polynucleotide represented by SEQ ID NO: 1 comprises at least one of the polynucleotides represented by SEQ ID NOs: 2-6;
and/or in (a4), the polynucleotide having at least 90% identity with the polynucleotide represented by SEQ ID NO: 13 comprises at least one of the polynucleotides represented by SEQ ID NOs: 14-18;
and/or in (a5), the polynucleotide encoding any one of the amino acid sequences having at least 90% sequence identity with the amino acid sequence represented by SEQ ID NO: 7 comprises at least one of the polynucleotides represented by SEQ ID NOs: 8-12.

3. A nucleic acid molecule composition, **characterised by** comprising the nucleic acid molecule of claim 1 or 2, and an additional R gene;
optionally the additional R gene comprises at least one gene selected from the group consisting of: Rpi-amr3i, Rpi-blb1, Rpi-sto1, Rpi-pta1, Rpi-blb2, Rpi-blb3, Rpi-abpt, R2-like, R2, Rpi-edn1.1, Rpi-edn1.2, Rpi-snk1.1, Rpi-snk1.2, Rpi-hjt1.1-Rpi-hjt1.3, Rpi-bt1, R1, R3a, R3b, Rpi-vnt1.1, Rpi-vnt1.2, Rpi-vnt1.3, Rpi-mcq1, Rpi-chc, Ph-3, or R8.

4. A biomaterial, **characterised in that** the biomaterial is any one selected from the following (b1) to (b3):
(b1) an expression cassette or expression cassette composition comprising the nucleic acid molecule of claim 1 or 2 or the nucleic acid molecule composition of claim 3;
(b2) a vector or vector composition comprising the nucleic acid molecule of claim 1 or 2 or the nucleic acid molecule composition of claim 3, or a vector or vector composition comprising the expression cassette or expression cassette composition of (b1); and
(b3) a host cell comprising the nucleic acid molecule of claim 1 or 2 or the nucleic acid molecule composition of claim 3, or a host cell comprising the expression cassette or expression cassette composition of (b1), or a host cell comprising the vector or vector composition of (b2); optionally, the host cell comprises bacterial or fungal cell.

5. The biomaterial according to claim 4, **characterised in that** the expression cassette or expression cassette composition further comprises an operatively connected promoter;
optionally the promoter comprises an operatively connected endogenous promoter and/or an operatively connected heterogeneous promoter.

6. A promoter, **characterised by** comprising at least one of the following polynucleotides: the polynucleotide represented by SEQ ID NO: 19, and/or a polynucleotide having at least 75% sequence identity with the polynucleotide represented by SEQ ID NO: 19.

7. The promoter according to claim 6, **characterised in that** the polynucleotide having at least 75% sequence identity with the polynucleotide represented by SEQ ID NO: 19 comprises at least one of the polynucleotides represented by SEQ ID NOs: 20-24.

8. A method for enhancing a plant's resistance to a plant disease caused by at least one race of at least one *Phytophthora sp.*, wherein the method comprises modifying at least one plant cell to comprise a heterologous polynucleotide comprising the nucleic acid molecule of claim 1 or 2 or the nucleic acid molecule composition of claim 3.

9. The method according to claim 8, **characterised in that** the method further comprises regenerating the plant cell into a plant whose genome comprises the heterologous polynucleotide.

10. A method for limiting a plant disease caused by at least one race of at least one *Phytophthora sp*. in crop production, **characterised in that** the method comprises:
modifying at least one plant cell to comprise a heterologous polynucleotide comprising the nucleic acid molecule of claim 1 or 2 or the nucleic acid molecule composition of claim 3;
regenerating the plant cell into a plant whose genome comprises the heterologous polynucleotide; and
growing seedlings, tubers, or seeds of the plant, and cultivating the seedlings, tubers, or seeds under conditions conducive to the growth and development of the plant.

11. A method for detecting disease resistance of a plant, **characterised in that** the method comprises detecting the presence of the nucleic acid molecule of claim 1 or 2 or the nucleic acid molecule composition of claim 3 in the plant, or in plant parts or plant cells thereof.

12. The method according to claim 11, **characterised in that** the method for detecting the presence of the nucleic acid molecule of claim 1 or 2 or the nucleic acid molecule composition of claim 3 comprises: detecting the presence of the nucleic acid molecule or nucleic acid molecule composition by detecting an intact nucleic acid molecule or nucleic acid molecule composition, or by detecting at least one molecular marker in the nucleic acid molecule or nucleic acid molecule composition.

13. The method according to claim 11 or 12, **characterised in that** the means of detecting the presence of the nucleic acid molecule of claim 1 or 2 or the nucleic acid molecule composition of claim 3 comprises PCR amplification, nucleic acid sequencing, nucleic acid hybridization, or an immunoassay for detecting a protein or polypeptide encoded by the nucleic acid molecule or the nucleic acid molecule composition.

14. The method according to any one of claims 8-13, **characterised in that** the plant comprises solanaceous plants;
preferably the solanaceous plants comprise, but are not limited to, at least one selected from the group consisting of: potato, tomato, eggplant, pepper, tobacco, petunia, tomatillo, and cape gooseberry;
more preferably the solanaceous plants are potato and/or tomato.

15. A method for selecting a plant, **characterised in that** the method comprises: detecting the presence of the nucleic acid molecule of claim 1 or 2 or the nucleic acid molecule composition of claim 3 in a plant, or in plant parts or plant cells thereof; and selecting a plant that comprises at least one copy of the nucleic acid molecule or nucleic acid molecule composition in its genome;
preferably the method comprises using the primers represented by SEQ ID NOs:25-38.

16. A protein, **characterised by** comprising at least one of the following amino acid sequences:
(e1) the amino acid sequence represented by SEQ ID NO: 7;
(e2) an amino acid sequence encoded by the polynucleotide represented by SEQ ID NO: 1 or a polynucleotide having at least 90% identity with the polynucleotide represented by SEQ ID NO: 1;
(e3) an amino acid sequence encoded by the polynucleotide represented by SEQ ID NO: 13 or a polynucleotide having at least 90% identity with the polynucleotide represented by SEQ ID NO: 13;
(e4) an amino acid sequence having at least 90% sequence identity with the amino acid sequence represented by SEQ ID NO: 7, wherein a protein comprising the amino acid sequence is capable of conferring resistance to a plant disease caused by at least one variety of at least one species of *Phytophthora*;
(e5) a fusion amino acid sequence obtained by connecting a tag to the N-terminus and/or C-terminus of the amino acid sequence represented by SEQ ID NO: 7; and
(e6) an amino acid sequence with the same function obtained by substituting and/or deleting and/or adding one or more amino acid residues in the amino acid sequence represented by SEQ ID NO: 7.

17. The protein according to claim 16, **characterised in that** in (e2), the polynucleotide having at least 90% identity with the polynucleotide represented by SEQ ID NO: 1 comprises at least one of the polynucleotides represented by SEQ ID NOs: 2-6; and/or
the polynucleotide having at least 90% identity with the polynucleotide represented by SEQ ID NO: 13 comprises at least one of the polynucleotides represented by SEQ ID NOs: 14-18; and/or
the amino acid sequence having at least 90% sequence identity with the amino acid sequence represented by SEQ ID NO: 7 comprises at least one of the polynucleotides represented by SEQ ID NOs: 8-12.

18. A plant or a plant part thereof, **characterised in that** the plant comprises a transgenic plant comprising the nucleic acid molecule of claim 1 or 2 or the nucleic acid molecule composition of claim 3.

19. A method for producing a plant, **characterised in that** the method comprises transforming a plant with at least one of the following materials: the nucleic acid molecule of claim 1 or 2, the nucleic acid molecule composition of claim 3, the expression cassette or expression cassette and the vector or vector composition of claim 4.
